(19) Europäisches Patentamt
European Patent Office
Office européen des brevets

(11) Veröffentlichungsnummer: **0 257 557 B1**

(12) # EUROPÄISCHE PATENTSCHRIFT

(45) Veröffentlichungstag der Patentschrift: **04.12.91**

(21) Anmeldenummer: **87112061.4**

(22) Anmeldetag: **20.08.87**

(51) Int. Cl.⁵: **C07C 49/175**, C07C 49/255, C07C 47/198, C07C 47/277, C07C 45/51, C07C 45/67, C07C 69/734, C07C 69/708, C07C 67/327

(54) **Verfahren zur Herstellung von bifunktionellen Verbindungen.**

(30) Priorität: **27.08.86 DE 3629119**
**16.01.87 DE 3701113**

(43) Veröffentlichungstag der Anmeldung:
**02.03.88 Patentblatt 88/09**

(45) Bekanntmachung des Hinweises auf die Patenterteilung:
**04.12.91 Patentblatt 91/49**

(84) Benannte Vertragsstaaten:
**BE CH DE FR GB LI NL**

(56) Entgegenhaltungen:
**DE-A- 2 300 638**
**DE-A- 3 602 253**
**DE-B- 1 031 777**

**SYNTHESIS, Nr. 6/7, Juni/Juli 1985, Seite 593, Nr. 2.2, Stuttgart, DE; "Partial or selective hydrolysis of malonaldehyde bis-acetals"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms 1(DE)**
Erfinder: **Hupfer, Leopold, Dr.**
**Waltershoehe 3**
**W-6701 Friedelsheim(DE)**

**EP 0 257 557 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Verfahren zur katalytischen Herstellung von bifunktionellen Verbindungen aus Tetraalkoxialkanen oder Dialkoxialkansäureestern.

Bifunktionelle Verbindungen sind in der Regel sehr wertvolle Bausteine in der organischen Synthese. Besonders interessant sind solche Verbindungen, die einfache Schutzgruppen oder Gruppierungen mit unterschiedlicher bzw. abgestufter Reaktivität besitzen, so daß mit jeder Funktion gezielte Umsetzungen möglich sind.

Zu dieser wichtigen Stoffklasse gehören beispielsweise bei den $C_3$-Verbindungen die Monoacetale des Malonaldehyds oder 3-Alkoxiacroleine (Synthesis, (1985), S. 592-595). Zur Herstellung dieser Verbindungen auf einem technisch gangbaren Wege scheiden Synthesevarianten, wie die Ozonolyse von 1,1-Dialkoxi-3-alkenen (C.A. (1961), 55, 20926) oder die Addition von Alkoholen an Propargylaldehyd (Skoldinov et al, Zh. Org. Chim. (1968), S. 183, und (1970), S. 422) aus, da die Vorprodukte nur schwer zugänglich sind.

Auf Basis des Acroleins sind 1,1,3-Trialkoxipropene herstellbar. Nachteilig ist hierbei, daß die Synthese der 1,1,3-Trialkoxipropene über mehrere Stufen erfolgen muß und daß man über eine partielle Hydrolyse der Acetale - nach einer vorgelagerten Halogenierung - nur die Halogenmalonaldehyd-monoacetale herstellen kann (US-PS 2 816 109, US-PS 2 815 384, US-PS 2 870 221, Price Moos, J. Am. chem. Soc., 67 (1945), S. 207, McElvain, Morris, J. Am. chem. Soc. 73 (1950), S. 206, Rothstein, Whiteley, Soc. (1953), S. 4015.

Eine weitere Möglichkeit für den Aufbau von Malonaldehydderivaten besteht in der Kombination von gängigen $C_1$- und $C_2$-Verbindungen. In der US-PS 2 465 586 wird die Herstellung solcher Verbindungen durch Kondensation von Ameisensäurederivaten mit Acetaldehydacetalen beschrieben, wobei die Kondensation allerdings in Gegenwart von großen Mengen an metallischem Natrium oder Natriumalkoholaten mit schlechten Ausbeuten verläuft.

Aus der DE-A- 10 31 777 ist die Batchfahrweise bekannt. Die Reaktion wird hier durch Ionentauscher (Styrol-Divinylbenzol-Basis) katalysiert. Die Verweilzeit beträgt 2 h.

Die partielle Hydrolyse von Tetraalkoxipropanen zu Monoacetalen des Malonaldehyds bzw. 3-Alkoxiacrolein (Breitmeier, Gassenmann, Chem. Ber. 104 (1971) , S. 665 - 667, Ruegg et al, Helv. chim. Acta 42 (1959), S. 847 - 853) führt in der Regel zu Mischungen, die sich nur schwer reinigen lassen, da die als Ausgangsprodukte benutzten Tetraalkoxipropane und die als Reaktionsprodukte entstehenden 3-Alkoxiacroleine fast identische Siedepunkte besitzen, wodurch eine destillative Trennung unmöglich wird (Eskenazi, Maitte, Bull. Soc. Chem. Fr. 1976, S. 995 - 8). Um zur reinen Verbindung zu kommen, muß man über die Alkalisalze der Enolverbindungen als Zwischenstufe gehen und diese dann durch Alkylierung oder Acylierung weiter derivatisieren (Eskenazi, Maitte, Bull. Soc. Chim. Fr. 1976, S. 995 - 8, Maddaluno, D'Angelo, Tetrahedron Letters, (1983), S. 895 - 898). Reine 3-Alkoxiacroleine erhält man, wenn Tetraalkoxipropane mit cyclischen Anhydriden, wie beispielsweise Maleinsäureanhydrid, Bernsteinsäureanhydrid, Glutarsäureanhydrid oder Phthalsäureanhydrid im Temperaturbereich von 150 bis 180 °C, erhitzt werden. Nachteilig an diesem Verfahren ist der Tatbestand, daß die cyclischen Anhydride in stöchiometrischer Menge benötigt werden und daß dieser Prozeß mit einem Zwangsanfall der entsprechenden Ester der Maleinsäure, Bernsteinsäure usw. gekoppelt ist.

Es bestand daher die Aufgabe, den zuvor genannten Nachteilen abzuhelfen.

Es wurde gefunden, daß man die Nachteile der bekannten Verfahren vermeidet und bifunktionelle Verbindungen der Formel (I)

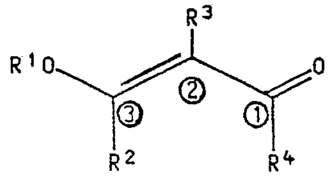

(I),

in der $R^1$, $R^2$ und $R^3$ Alkylreste mit 1 bis 6 Kohlenstoffatomen, $R^2$ und $R^3$ zusätzlich auch Wasserstoff, $R^2$ zusätzlich auch Alkoxireste und $R^4$ Wasserstoff, Alkylreste mit 1 bis 6 Kohlenstoffatomen und Alkoxireste bedeuten können erhält, wenn man Tetraalkoxialkane der Formel II oder Dialkoxialkansäureester der Formel III

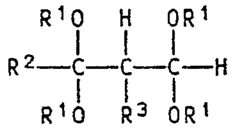 (II) 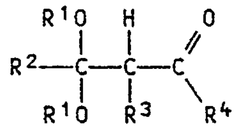 (III)

in der $R^1$ bis $R^4$ obige Bedeutung haben, in Gegenwart von Zeolithen und/oder Phosphaten als Katalysatoren umsetzt.

Die Herstellung der als Ausgangsstoffe verwendeten Tetraalkoxialkane und Dialkoxialkansäureester ist z.B. aus US-PS 2 459 076 bekannt.

Als Katalysatoren im Sinne der Erfindung sind im allgemeinen geeignet Zeolithe des Pentasiltyps wie Aluminiumsilikatzeolithe, Borosilikatzeolithe, Eisensilikatzeolithe, und Zeolithe des Faujasit-Typs.

Die Zeolithe können z.B. mit Alkalimetallen, Übergangsmetallen und mit seltenen Erdmetallen dotiert werden.

Man kann als Katalysatoren aber auch Phosphate der Elemente B, Al, Zr, Fe, Sr oder deren Gemische verwenden. Auch hydrothermal hergestellte Phosphate sind z.B. als Katalysatoren geeignet z.B. hydrothermal hergestellte Aluminiumphosphate, Siliciumaluminiumphosphate oder Siliciumeisenaluminiumphosphate. Daneben kann man als Katalysatoren Phosphorsäure auf Trägermaterialien verwenden.

Als Katalysatoren für das erfindungsgemäßen Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustauch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit-bzw. Chabasit-Typ oder Zeolithe vom Faujasit-Typ, z.B. Y-, X- oder L-Zeolithe. In dieser Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch eine hohes $SiO_2/Al_2O_3$-Verhältnis gekennzeichnet sowie durch Porengrößen, die zwischen denen der Zeolithe vom Typ A und denen vom Type X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alimino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid im wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Diese Aluminosilikatzeolithe können in etherischem Medium wie Diethylenglykoldimethylether, in alkoholischem Medium wie Methanol bzw. 1,4-Butandiol oder in Wasser synthetisiert werden.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid

in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200° C unter autogenem Druck.

Zu den erfindungsgemäß verwendbaren siliciumreichen Zeolithen ($SiO_2/Al_2O_3 \geqq 10$) gehören auch die verschiedenen ZSM-Typen, Ferrierit, NU-1 und Silicalit®.

Die so hergestellten Alumino-, Boro- und Eisensilikatzeolithe können nach ihrer Isolierung, Trocknung bei 100 bis 160° C, vorzugsweise 110° C und Calcinierung bei 450 bis 550° C, vorzugsweise 500° C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110° C/16 h getrocknet und bei 500° C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschießende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550° C, bevorzugt 500° C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100° C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Ni(NO_3)_2 \times 6\ H_2O$ oder $Ce(NO_3)_3 \times 6\ H_2O$ oder $La(NO_3)_3 \times 6\ H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird am Rotationsverdampfer von Wasser befreit. Danach wird der getränkte Zeolith bei etwa 150° C getrocknet und bei etwa 550° C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100° C unter Rühren etwa 24 h aufzuschlämmen. Nach Abfiltrieren, Trocknen bei etwa 150° C und Calcinierung bei etwa 500° C kann das so

gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80° C im Kreislauf 15 bis 20 h leitet. Danach wird mit Wasser ausgewaschen, bei etwa 150° C getrocknet und bei etwa 550° C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80° C behandelt. Nach der Behandlung wird mit Wasser gewaschen, bei 110° C/16 Stunden getrocknet und bei 500° C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80° C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400° C bis 500° C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160 °C, getrocknet und bei Temperaturen von im allgemeinen 450° C bis 600° C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90° C, vorzugsweise 60 bis 80° C, über einen Zeitraum von 0,5 bis 5, vorzugsweise mit 12 bis 20 gew.-%iger Salzsäure, behandelt. Zweckmäßig wird das zeolithische Material anschließend ausgewaschen, bei Temperaturen von 100 bis 160° C getrocknet und bei Temperaturen von 450 bis 600° C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren.

Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110° C getrocknet und bei 500° C calciniert.

Weitere Katalysatoren für die Herstellung von bifunktionellen Verbindungen der Formel (I) aus Tetraalkoxialkanen der Formel (II) oder Dialkoxialkansäureester der Formel (III) sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische.

Als Aluminiumphosphat-Katalysatoren werden für das erfindungsgemäße Verfahren, insbesondere unter hydrothermalen Bedingungen, synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

Beispielsweise $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei etwa 150° C 20 bis 60 h unter autogenem Druck in einem Autoklaven umsetzt. Das abfiltrierte $AlPO_4$ wird getrocknet bei 100 bis 160° C und calciniert bei 450 bis 550° C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200° C unter autogenem Druck während 200 bis 400 h synthetisiert. Verwendet man anstelle DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidon-Lösung bei 150 bis 200° C unter autogenem Druck während 50 bis 200 h.

Für das erfindungsgemäße Verfahren kann man auch bekannte Siliciumaluminiumphosphate wie SAPO-5, SAPO-11, SAPO-31 und SAPO-34 einsetzen. Diese Verbindungen werden hergestellt durch Kristallisation aus wäßriger Mischung bei 100 bis 250° C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200° C, während 20 bis 200 h unter autogenem Druck in einem Rührautoklaven erhalten. Die Trocknung des abfiltrierten Pulvers erfolgt bei 110 bis 160° C und die Calcination bei 450 bis 550° C.

Borphosphate als Katalysatoren für das erfindungsgemäße Verfahren kann man beispielsweise durch Mischen und Kneten von konzentrierter Borsäure und Phosphorsäure und durch anschließende Trocknung und Calcination in Inertgas-, Luft- oder Dampfatmosphäre bei 250 bis 650° C vorzugsweise 300 bis 500° C herstellen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung in der Regel gewählten Reaktionsbedingungen sind in der bevorzugten Gasphase 100 bis 500° C, insbesondere 200 bis 400° C, und eine Belastung WHSV = 0,1 bis 20 $h^{-1}$, insbesondere 0,5 bis 5 $h^{-1}$ (g Einsatzgemisch/g Katalysator und Stunde).

Es ist auch möglich, die Reaktion in der Flüssigphase, z.B. Suspensions-, Riesel- oder Sumpffahrweise, bei Temperaturen zwischen 50 und 200° C durchzuführen.

Das Verfahren wird im allgemeinen bei Normaldruck oder je nach Flüchtigkeit der Ausgangsverbindung bei vermindertem oder erhöhtem Druck durchgeführt, wobei die Durchführung kontinuierlich und diskontinuierlich erfolgen kann.

Schwerflüchtige oder feste Ausgangsstoffe werden in gelöster Form, z.B. in THF-, Toluol- oder Petrolether-Lösung eingesetzt. Generell ist eine Verdünnung des Ausgangsstoffes mit derartigen Lösungsmitteln oder mit Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetztes Einsatzgemisch wird gegebenenfalls, wenn nötig, in die Umsetzung zurückgeführt.

Besonders vorteilhaft ist es, die gasförmigen Reaktionsprodukte sofort in eine Trennung einzubringen und in die Einzelkomponenten zu zerlegen. Eine derartige Trennung kann z.B. in einer Fraktionierkolonne durchgeführt werden. Durch die Trennung kann eine Rückreaktion unterbunden und ein hoher Umsatz erzielt werden.

Beispiele 1 bis 17

Die Reaktionen in der Gasphase werden unter isothermen Bedingungen in einem Rohrreaktor (Wendel, 0,6 cm Innendurchmesser, 90 cm Länge) im Verlauf von mindestens 6 Stunden durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch. Die GC-Analysen erfolgen nach 6 Stunden.

Die jeweils eingesetzten Katalysatoren sind:

Katalysator A

Ein Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50 : 50 Gew.-%) bei 170° C unter autogenem Druck in einem Rührautoklaven hergestellt. Nach Abfiltrieren und Auswaschen wird das kristalline Reaktionsprodukt bei 100° C/24 h getrocknet und bei 500° C/24 h calciniert. Dieser Borosilikatzeolith setzt sich zusammen aus 94,2 Gew.-% $SiO_2$ und 2,3 Gew.-% $B_2O_3$.

Mit diesem Material werden durch Verformen mit einem Verformungshilfsmittel 2-mm-Stränge hergestellt, die bei 100° C/16 h getrocknet und bei 500° C/24 h calciniert werden.

Katalysator B

Ein Aluminosilikatzeolith vom Pentasil-Typ wird unter hydrothermalen Bedingungen bei autogenem Druck und 150° C aus 65 g hochdispersem $SiO_2$, 20,3 g $Al_2(SO_4)_3 \times 18\ H_2O$ in 1 kg einer wäßrigen 1,6-Hexandiamin-Lösung (Mischung 50 : 50 Gew.-%) in einem Rührautoklaven hergestellt. Nach Abfiltrieren und Auswaschen wird das kristalline Reaktionsprodukt bei 110° C/24 h getrocknet und bei 500° C/24 h calciniert. Dieser Aluminosilikatzeolith enthält 91,6 Gew.-% $SiO_2$ und 4,6 Gew.-% $Al_2O_3$. Der Katalysator wird zu 2-mm-Strängen verformt, bei 110° C/16 h getrocknet und bei 500° C/24 h calciniert.

Katalysator C

Katalysator C wird erhalten, indem man die Stränge des Katalysators A mit einer wäßrigen $Cs_2CO_3$-Lösung imprägniert, danach bei 130° C/2 h trocknet und bei 540° C/2 h calciniert. Der Cs-gehalt beträgt 0,6 Gew.-%

Katalysator D

200 g Katalysator A werden mit 1 l einer wäßrigen Lösung aus 16,7 g $FeCl_3$ x 6 $H_2O$ und 50 g $NH_4Cl$ 24 h bei Raumtemperatur ionenausgetauscht, danach gründlich mit $H_2O$ Cl-frei gewaschen, bei 150° C/1 h getrocknet und bei 500° C/2 h calciniert. Dieses Pulver wird mit hochdispersem $SiO_2$ im Gewichtsverhältnis 70 : 30 verformt. Nach Trocknung werden die Stränge bei 500° C/16 h calciniert.

Katalysator E

Katalysator E wird erhalten, indem man Stränge des Katalysators A mit einer wäßrigen Lösung aus Cer- und Palladiumnitrat imprägniert, danach bei 130° C/2 h trocknet und bei 540° C/2 h calciniert. Der Ce-gehalt beträgt 2,3 Gew.-%, der Pd-gehalt 0,5 Gew.-%.

Katalysator F

Katalysator F wird wie Katalysator C hergestellt, wobei jedoch $Cs_2CO_3$ durch $Fe(NO_3)_3$ ersetzt wird. Der Fe-gehalt beträgt 2,9 Gew.-%.

Katalysator G

Katalysator G wird wie Katalysator C hergestellt, wobei jedoch $Cs_2CO_3$ durch $Ni(NO_3)_2$ ersetzt wird. Der Ni-gehalt beträgt 3,3 Gew.-%.

Katalysator H

Katalysator H wird wie Katalysator C hergestellt, wobei jedoch $Cs_2CO_3$ durch $Ce(NO_3)_3$ ersetzt wird. Der Ce-gehalt beträgt 1,65 Gew.-%.

Katalysator I

Katalysator I wird wie Katalysator C hergestellt, wobei jedoch $C2_2CO_3$ durch $Co(NO_3)_2$ ersetzt wird. Der Co-gehalt beträgt 3,1 Gew.-%.

Katalysator J

Katalysator J wird wie Katalysator C hergestellt, wobei jedoch $Cs_2CO_3$ durch $Cr(NO_3)_3$ ersetzt wird. Der Cr-gehalt beträgt 1,9 Gew.-%.

Katalysator K

$AlPO_4$-$_{12}$ (APO-$_{12}$) wird synthetisiert, indem man 200 g 98 %ige Phosphorsäure und 136 g Boehmit in 400 g Wasser löst bzw. suspendiert, hierzu eine wäßrige Lösung aus 60 g Ethylendiamin und 320 g $H_2O$ zugibt und diese Mischung in einem Rührautoklaven bei 200° C während 24 Stunden unter autogenem Druck umsetzt. Nach Abfiltrieren wird das kristalline Material bei 120° C getrocknet und bei 500° C/16 h calciniert. Das so synthetisierte $AlPO_4$-12 enthält 55,5 Gew.-% $P_2O_5$, 39,7 Gew.-% $Al_2O_3$. Dieses Material wird mit Verstrangungshilfsmitteln zu 3-mm-Strängen verformt, abermals bei 120° C getrocknet und bei 500° C/6 h calciniert.

Katalysator L

Siliciumaluminiumphosphat-5 (SAPO-5) wird hergestellt aus einer Mischung aus 220 g 98 %ige Phosphorsäure, 136 g Boehmit, 60 g Kieselsol (30 %ig) 287 g Tripropylamin und 587 g $H_2O$. Diese Mischung wird bei 150° C während 168 Stunden unter autogenem Druck umgesetzt. Nach Filtration wird das kristalline Produkt bei 120° C getrocknet und bei 500° C calciniert. SAPO-5 enthält 49,8 Gew.-%

$P_2O_5$, 33,0 Gew.-% $Al_2O_3$, 6,2 Gew.-% $SiO_2$. SAPO-5 wird mit einem Verstrangungshilfsmittel zu 3-mm-Strängen verformt, bei 120° C getrocknet und bei 500° C calciniert.

Katalysator M

Im Handel erhältliches Zirkonphosphat $Zr_3(PO_4)_4$ in Reinsubstanz verformt.

Katalysator N

Im Handel erhältlicher L-Zeolith (Baylith L®) wird mit Boehmit im Gewichtsverhältnis 80 : 20 zu 2-mm-Strängen verformt. Nach Trocknung bei 110° C/16 h und Calcination bei 500° C/16 h liegt der fertige Katalysator N vor.

Katalysator O

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120°C getrocknet und bei 450°C calciniert. Katalysator O enthält 47,1 Gew.-% Ce und 12,7 Gew.-% P.

Beispiel 18

Es werden 200 ml/h 1,1,3,3-Tetramethoxipropan in einem Stickstoffstrom von 300 ml/h verdampft und bei einer Temperatur von 220° C über 1 l eines Borzeolithkatalysators A, der in einem von außen elektrisch beheizten Reaktionsrohr untergebracht ist, geleitet.

Das gasförmige Reaktionsgemisch wird in den Mittelteil einer Fraktionierkolonne geleitet, das gebildete Methanol sowie andere Leichtsieder destillieren über Kopf ab, während 3-Methoxiacrolein mit geringen Mengen nicht umgesetztem Ausgangsprodukt im Sumpf der Kolonne abgezogen werden können.

Die Reinherstellung gelingt in einfacher Weise durch eine übliche Destillation.

Der Umsatz von 1,1,3,3-Tetramethoxipropan ist >95 %, die Destillationsausbeute beträgt 91 %.

Der Katalysator A zeigt nach 85 Stunden Reaktionszeit noch keine Deaktivierungserscheinungen.

Beispiel 19

Es wird wie im Beispiel 18 verfahren, jedoch 1,1,3,3-Tetraethoxipropan zur Reaktion gebracht.

Die Ausbeute an 3-Ethoxipropenal beträgt 82 % bei einem Umsatz von 93 %.

Beispiel 20

Es wird analog Beispiel 18 gearbeitet, jedoch das Reaktionsprodukt nicht in einer Kolonne, sondern in eine Vorlage geleitet, die mit auf mind. pH 10 eingestelltem Methanol beschickt ist. Der Umsatz des eingesetzten 1,1,3,3-Tetramethoxipropans liegt >95 %, die Ausbeute an 3,3-Dimethoxipropionaldehyd beträgt 61 % d. Th.

Beispiel 21

Beispiel 21 bezieht sich wie Beispiel 20 auf die Herstellung von 3,3-Dimethoxipropionaldehyd.

In einer Rührapparatur wird zu 700 ml Methanol, die mit 10,3 g 30 %iger methanolischer Natriummethylatlösung auf pH 12 gestellt werden, eine Lösung von 295 g 3-Methoxiacrolein in 175 ml Tetrahydrofuran bei 25°C langsam zugetropft und nach beendeter Zugabe 3 Stunden lang bei 25°C nachgerührt. Anschließend wird mit verdünnter Schwefelsäure auf pH 7 gestellt und aufgearbeitet. Es werden hierbei 256 g 3,3-Dimethoxipropionaldehyd (Kp = 59°C/22 mbar) erhalten. Ausbeute: 63 % d. Th.

Beispiel 22

Es wird analog Beispiel 18 gearbeitet und 2,2,4,4-Tetramethoxipentan zu 4-Methoxi-pent-3-en-2-on umgesetzt, wobei der Umsatz >95 % und die Ausbeute 93 % beträgt.

Beispiel 23

Es wird analog Beispiel 18 gearbeitet und 3,3-Dimethoxibuttersäuremethylester zu 3-Methoxicrotonsäureester umgesetzt, wobei der Umsatz >95 % und die Ausbeute bei 96 % liegt.

Tabelle 1

| Beispiel | 1 | 2 | 3 | 4 | 5 |
|---|---|---|---|---|---|
| Edukt | 1,1,3,3-Tetramethoxipropan mit $H_2O$ gesättigt (~ 4 Gew.-%) | | | | |
| Katalysator | B | C | D | E | M |
| Temperatur | 300°C | 300°C | 300°C | 275°C | 300°C |
| WHSV | 2 $h^{-1}$ | 2 $h^{-1}$ | 2 $h^{-1}$ | 2 $h^{-1}$ | 2 $h^{-1}$ |
| Umsatz % | 57,8 | 81,8 | 98,8 | 64,1 | 52,4 |
| Selektivität % | | | | | |
| Methoxi-acrolein | 98,3 | 93,0 | 95,7 | 94,5 | 90,5 |

Fortsetzung Tabelle 1

| Beispiel | 6 | 7 | 8 | 9 |
|---|---|---|---|---|
| Edukt | 1,1,3,3-Tetramethoxipropan mit $H_2O$ gesättigt (~ 4 Gew.-%) | | | |
| Katalysator | K | L | N | O |
| Temperatur | 300°C | 300°C | 350°C | 300°C |
| WHSV | 2,5 $h^{-1}$ | 2,5 $h^{-1}$ | 2,5 $h^{-1}$ | 2,5 $h^{-1}$ |
| Umsatz % | 7,0 | 10,9 | 10,3 | 15,5 |
| Selektivität % | | | | |
| Methoxi-acrolein | 94,3 | 73,4[1)] | 86,4 | 94,8 |

1) plus 3,3-Dimethoxipropanal mit 10 % Selektivität

Tabelle 2

| Beispiel | 10 | 11 | 12 | 13 | 14 |
|---|---|---|---|---|---|
| Edukt | 1,1,3,3-Tetramethoxipropan : THF = 75 : 25 Gew-.% | | | | |
| Katalysator | F | G | H | I | J |
| Temperatur | 300°C | 300°C | 300°C | 300°C | 300°C |
| WHSV | 2 $h^{-1}$ | 2 $h^{-1}$ | 2 $h^{-1}$ | 2 $h^{-1}$ | 2 $h^{-1}$ |
| Umsatz % | 97,4 | 80,1 | 61,2 | 44,9 | 44,1 |
| Selektivität % | | | | | |
| Methoxi-acrolein | 82,8 | 87,3 | 94,8 | 93,4 | 94,5 |

Tabelle 3

| Beispiel | 15 | 16 | 17 |
|---|---|---|---|
| Edukt | 3,3-Dimethoxipropionsäure-methylester : THF = 50 : 50 Gew.% | | |
| Katalysator | A | A | B |
| Temperatur | 200°C | 300°C | 300°C |
| WHSV | 2 $h^{-1}$ | 2,5 $h^{-1}$ | 2,5 $h^{-1}$ |
| Umsatz % | 100 | 100 | 100 |
| Selektivität % | | | |
| 3-Methoxiacrylsäuremethylester | 95,5 | 91,7 | 92,6 |

## Patentansprüche

1. Verfahren zur Herstellung von bifunktionellen Verbindungen der Formel (I)

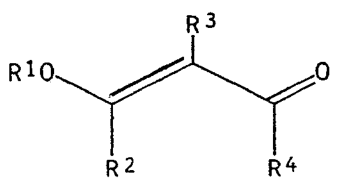

(I),

in der $R^1$, $R^2$ und $R^3$ Alkylreste mit 1 bis 6 Kohlenstoffatomen, $R^2$ und $R^3$ zusätzlich auch Wasserstoff, $R^2$ zusätzlich auch Alkoxireste und $R^4$ Wasserstoff, Alkylreste mit 1 bis 6 Kohlenstoffatomen und Alkoxireste bedeuten können dadurch gekennzeichnet, daß man Tetraalkoxialkane der Formel (II) oder Dialkoxialkansäureester der Formel (III)

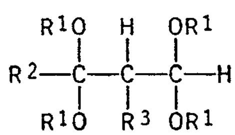

(II)

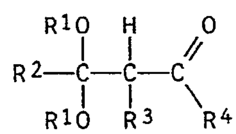

(III)

in der $R^1$ bis $R^4$ obige Bedeutung haben, in Gegenwart von Zeolithen und/oder Phosphaten als Katalysatoren umsetzt.

2. Verfahren nach Anspruch 1, dadurch gekennzeichnet, daß man als Ausgangsstoff 1,1,3,3-Tetramethoxipropan oder 2,2,4,4-Tetramethoxipentan einsetzt.

3. Verfahren nach Anspruch 1, dadurch gekennzeichnet, daß man als Ausgangsstoff 3,3-Dimethoxipropionsäuremethylester oder 3,3-Dimethoxibuttersäuredimethylester einsetzt.

4. Verfahren nach Anspruch 1, dadurch gekennzeichnet, daß man als Katalysatoren Zeolithe des Pentasil-Typs verwendet.

5. Verfahren nach Anspruch 1 und 4, dadurch gekennzeichnet, daß man als Katalysatoren Aluminiumsilikatzeolithe verwendet.

6. Verfahren nach Anspruch 1 und 4, dadurch gekennzeichnet, daß man als Katalysatoren Borsilikatzeolithe verwendet.

7. Verfahren nach Anspruch 1 und 4, dadurch gekennzeichnet, daß man als Katalysatoren Eisensilikatzeolithe verwendet.

8. Verfahren nach Anspruch 1, dadurch gekennzeichnet, daß man als Katalysatoren Zeolithe des Faujasit-Typs verwendet.

9. Verfahren nach Anspruch 1, dadurch gekennzeichnet, daß man mit Alkalimetallen, Übergangsmetallen, seltenen Erdmetallen dotierte Zeolithe als Katalysatoren verwendet.

10. Verfahren nach Anspruch 1, dadurch gekennzeichnet, daß man als Katalysatoren Phosphate der Elemente B, Al, Zr, Fe, Sr oder deren Gemische verwendet.

11. Verfahren nach Anspruch 1, dadurch gekennzeichnet, daß man als Katalysatoren hydrothermal hergestellte Phosphate verwendet.

12. Verfahren nach Anspruch 1, dadurch gekennzeichnet, daß man als Katalysatoren hydrothermal hergestellte Aluminiumphosphate oder Siliciumaluminiumphosphate oder Siliciumeisenaluminiumphosphate verwendet.

## Claims

1. A process for the preparation of a bifunctional compound of the formula (I)

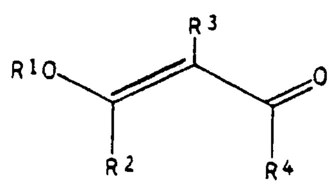

(I)

where $R^1$, $R^2$ and $R^3$ are each alkyl of 1 to 6 carbon atoms, $R^2$ and $R^3$ may furthermore be hydrogen, $R^2$ may furthermore be alkoxy and $R^4$ is hydrogen, alkyl of 1 to 6 carbon atoms or alkoxy, wherein a tetraalkoxyalkane of the formula (II) or a dialkoxyalkanoate of the formula (III)

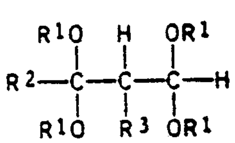

(II)

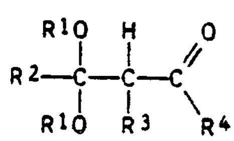

(III)

where $R^1$, $R^2$, $R^3$ and $R^4$ have the above meanings, is converted in the presence of a zeolite and/or a phosphate as catalysts.

2. A process as claimed in claim 1, wherein the starting material used is 1,1,3,3-tetramethoxypropane or 2,2,4,4-tetramethoxypentane.

3. A process as claimed in claim 1, wherein the starting material used is methyl 3,3-dimethoxypropionate

or dimethyl 3,3-dimethoxybutyrate.

4. A process as claimed in claim 1, wherein the catalyst used is a zeolite of the pentasil type.

5. A process as claimed in claim 1 and 4, wherein the catalyst used is an aluminosilicate zeolite.

6. A process as claimed in claim 1 and 4, wherein the catalyst used is a borosilicate zeolite.

7. A process as claimed in claim 1 and 4, wherein the catalyst used is an iron silicate zeolite.

8. A process as claimed in claim 1, wherein the catalyst used is a zeolite of the faujasite type.

9. A process as claimed in claim 1, wherein the catalyst used is a zeolite doped with an alkali metal, a transition metal or a rare earth metal.

10. A process as claimed in claim 1, wherein the catalyst used is a phosphate of the element B, Al, Zr, Fe or Sr, or a mixture of these.

11. A process as claimed in claim 1, wherein the catalyst used is a phosphate prepared by a hydrothermal method.

12. A process as claimed in claim 1, wherein the catalyst used is an aluminum phosphate, a silicon aluminum phosphate or a silicon iron aluminum phosphate prepared by a hydrothermal method.

## Revendications

1. Procédé de préparation de composés bifonctionnels de formule (I)

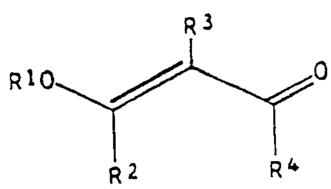

(I),

dans laquelle $R^1$, $R^2$ et $R^3$ peuvent représenter des restes alkyle à 1-6 atomes de carbone, $R^2$ et $R^3$ peuvent en plus représenter des atomes d'hydrogène, $R^2$ peut en plus représenter des restes alcoxy et $R^4$ peut représenter un atome d'hydrogène, des restes alkyle à 1-6 atomes de carbone et des restes alcoxy, caractérisé en ce qu'on fait réagir des tétraalcoxyalcanes de formule (II) ou des esters d'acide dialcoxyalcanoïque de formule (III)

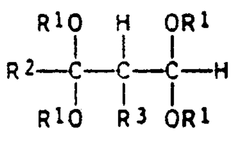

(II)

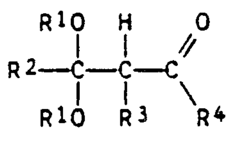

(III)

dans lesquelles $R^1$ à $R^4$ ont les significations précédentes, en présence de zéolithes et/ou de phosphates servant de catalyseurs.

2. Procédé selon la revendication 1, caractérisé en ce qu'on utilise, comme substance de départ, du 1,1,3,3-tétraméthoxypropane ou du 2,2,4,4-tétraméthoxypentane.

3. Procédé selon la revendication 1, caractérisé en ce qu'on utilise, comme substance de départ, de l'ester méthylique d'acide 3,3-diméthoxypropionique ou de l'ester méthylique d'acide 3,3-diméthoxybutyrique.

4. Procédé selon la revendication 1, caractérisé en ce qu'on utilise, comme catalyseurs, des zéolithes du type pentasil.

5. Procédé selon les revendications 1 et 4, caractérisé en ce qu'on utilise, comme catalyseurs, des zéolithes d'aluminosilicate.

6. Procédé selon les revendications 1 et 4, caractérisé en ce qu'on utilise, comme catalyseurs, des zéolithes de borosilicate.

7. Procédé selon les revendications 1 et 4, caractérisé en ce qu'on utilise, comme catalyseurs, des zéolithes de ferrosilicate.

8. Procédé selon la revendication 1, caractérisé en ce qu'on utilise, comme catalyseurs, des zéolithes du type faujasite.

9. Procédé selon la revendication 1, caractérisé en ce qu'on utilise, comme catalyseurs, des zéolithes dopées avec des métaux alcalins, des métaux de transition ou des métaux des terres rares.

10. Procédé selon la revendication 1, caractérisé en ce qu'on utilise, comme catalyseurs, des phosphates des éléments B, Al, Zr, Fe, Sr ou des mélanges de ces phosphates.

11. Procédé selon la revendication 1, caractérisé en ce qu'on utilise, comme catalyseurs, des phosphates préparés par synthèse hydrothermale.

12. Procédé selon la revendication 1, caractérisé en ce qu'on utilise, comme catalyseurs, des phosphates d'aluminium, des phosphates de silicium-aluminium ou des phosphates de silicium-fer-aluminium préparés par synthèse hydrothermale.